# EUROPEAN PATENT APPLICATION

(11) **EP 4 233 849 A1**
(43) Date of publication of application: **30.08.2023**
(21) Application number: 23158292.5
(22) Date of filing: 23.02.2023
(51) Int. Cl.: A61K 9/48, A61K 31/454

(54) **PHARMACEUTICAL COMPOSITION COMPRISING POMALIDOMIDE**

(30) Priority: 25.02.2022 SI 202200025
(71) Applicant: KRKA, d.d., Novo mesto, 8501 Novo mesto (SI)
(72) Inventor: PISANEC, Ga per, 8501 Novo mesto (SI); KORASA, Klemen, 8501 Novo mesto (SI)
(74) Representative: Kutzenberger Wolff & Partner

(57) **Abstract**

The invention pertains to pharmaceutical compositions and pharmaceutical dosage forms containing Pomalidomide, optionally in combination with one or more other active pharmaceutical substances, and low-substituted hydroxypropyl cellulose. The pharmaceutical compositions and pharmaceutical dosage forms are physically and chemically stable and are easy to manufacture. The pharmaceutical compositions and pharmaceutical dosage are provided in a solid form suitable for oral administration. The invention further provides a methods for making the pharmaceutical compositions and pharmaceutical dosage forms.

## Description

Priority is claimed of Slovenian patent application no. P-2022000025 that was filed on February 25, 2022.

The invention pertains to pharmaceutical compositions and pharmaceutical dosage forms containing Pomalidomide, optionally in combination with one or more other active pharmaceutical substances, and low-substituted hydroxypropyl cellulose. The pharmaceutical compositions and pharmaceutical dosage forms are physically and chemically stable and are easy to manufacture. The pharmaceutical compositions and pharmaceutical dosage are provided in a solid form suitable for oral administration. The invention further provides a methods for making the pharmaceutical compositions and pharmaceutical dosage forms.

Pomalidomide is used for the treatment of cancer. In combination with bortezomib and dexamethasone it is indicated in the treatment of adult patients with multiple myeloma. In combination with dexamethasone it is indicated in the treatment of adult patients with relapsed and refractory multiple myeloma.

Pomalidomide is available on the market in the form of hard capsules containing 1 mg, 2 mg, 3 mg and 4mg Pomalidomide per capsule. The capsules sold under trade name Imnovid contain Pomalidomide, mannitol, pregelatinized starch and sodium stearyl fumarate.

The chemical name of Pomalidomide is (RS) 4-Amino-2-(2,6-dioxopiperidin-3-yl)isoindole-1,3-dione. Pomalidomide has the following structure

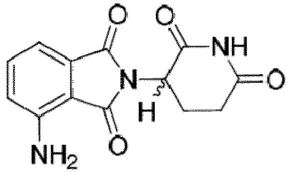

Pomalidomide has one stereochemical center. The active substance is provided as a racemic mixture, no optical rotation is observed.

Pomalidomide is a crystalline non-hygroscopic yellow powder, which is slightly soluble in acetone, acetonitrile, methylene chloride, methyl ethyl ketone and tetrahydrofuran; very slightly soluble in absolute ethanol, ethyl acetate, heptane, methanol, 2-propanol and toluene; and practically insoluble in water.

Pomalidomide is, according to BCS classification, classified as class IV, having low permeability and low solubility. BCS class IV drugs exhibit many characteristics that are problematic for effective oral and per oral delivery. Some of the problems associated include low aqueous solubility, poor permeability, erratic and poor absorption, inter and intra subject variability and a significant positive food effect which leads to low and variable bioavailability. Formulation and development of an efficacious delivery system for BCS class IV drugs are extremely difficult tasks for any formulator.

As reported in the European Public Assessment Report (EPAR) Pomalidomide shows instability problems at accelerated and room temperature conditions in the presence of particular excipients such as anhydrous lactose, microcrystalline cellulose, anhydrous dibasic calcium phosphate, croscarmellose sodium, and magnesium stearate.

Prior art documents already provide some compositions of Pomalidomide.

WO 2010 135396 discloses immediate release formulation in the form of a capsule or a tablet which comprises Pomalidomide and pharmaceutically acceptable excipient selected from starch, mannitol, or a mixture thereof. Particularly preferred is the formulation comprising a mixture of pregelatinized starch and spray dried mannitol.

CN 108 014 080 discloses a Pomalidomide pellet tablet and preparation method thereof.

WO 2018 024646 describes a capsule comprising Pomalidomide, maltodextrin and a filler selected from microcrystalline cellulose and calcium lactate.

WO 2019 185862 discloses a pharmaceutical composition comprising Pomalidomide in crystalline form A, a stabilizer selected from maltitol, sucrose, lactitol and mixtures thereof, wherein the composition comprises less than 50 wt.-% of inorganic phosphates, lactose and microcrystalline cellulose. It was found that presence of maltitol, lactitol and sucrose improves stability, whereas the presence of phosphates causes instability. It was also shown that the presence of microcrystalline cellulose causes incomplete dissolution.

WO 2021 209919 discloses formulations comprising anhydrous lactose as a diluent and hydroxypropyl methyl cellulose as a binder. Further excipients present in the composition are microcrystalline cellulose, croscarmellose sodium and magnesium stearate. It is stated that the compositions are stable when submitted to various standard conditions (accelerated and long term conditions) and exhibit high dissolution rate. For comparative examples without hydroxypropyl methyl cellulose it is shown that high dissolution profile cannot be achieved.

EP 3 900 701 A1 discloses a pharmaceutical composition which comprises: Pomalidomide or a pharmaceutically acceptable salt or solvate thereof; at least one filler which is isomalt; at least one binder which is pregelatinized starch; at least one lubricant which is sodium stearyl fumarate. It is stated that the composition has an improved final dissolution amount in neutral to medium acidic media and excellent stability.

As evident from the prior art, there appears some contradictory information about the influence of particular excipients on the properties of the pharmaceutical compositions comprising Pomalidomide. The prior art shows that Pomalidomide is susceptible to interaction with excipients and to degradation, and further it is shown that excipients influence dissolution profile, which is of extreme importance for the drug with low aqueous solubility. For these reasons, there remains a need for pharmaceutical compositions exhibiting a desired physical and chemical stability and exhibiting at the same time high dissolution profile and content uniformity.

It is thus an object according to the invention to provide pharmaceutical compositions and pharmaceutical dosage forms exhibiting a desired physical and chemical stability and exhibiting at the same time high dissolution profile and content uniformity.

This object has been achieved by the subject-matter of the patent claims.

A first aspect of the invention relates to a pharmaceutical composition, preferably for oral administration, comprising
- Pomalidomide or a pharmaceutically acceptable salt thereof;
- low substituted hydroxypropyl cellulose (L-HPC); and
- optionally at least one pharmaceutically acceptable excipient selected from the group consisting of binders, diluents, disintegrants, glidants and lubricants.

A second aspect of the invention relates to a pharmaceutical dosage form for oral administration comprising the pharmaceutical composition according to the invention.

In accordance with the invention, pharmaceutical compositions and pharmaceutical dosage forms for oral administration are provided designed for immediate release (conventional release) comprising
- Pomalidomide or a pharmaceutically acceptable salt thereof; and
- a pharmaceutically acceptable excipient which is low substituted hydroxypropyl cellulose (L-HPC).

In particular, there is provided a pharmaceutical composition, preferably for oral administration, (preferably capsule) comprising Pomalidomide, low substituted hydroxypropyl cellulose (L-HPC), and at least one pharmaceutically acceptable excipient selected from the group comprising at least one diluent, at least one disintegrant, at least one glidant and at least one lubricant.

Optionally, the at least one pharmaceutically acceptable excipient comprises diluent selected from starch or sugar alcohol and optionally one or more further diluents, optionally at least one disintegrant, optionally at least one glidant, and optionally at least one lubricant.

The pharmaceutical composition and pharmaceutical dosage form according to the invention exhibits excellent chemical and physical stability, it is stable under normal storage conditions and at the same time it provides an improved content uniformity.

The pharmaceutical composition and pharmaceutical dosage form according to the invention exhibits high stability on storage which is not limited by physical or chemical instability of active substance(s) or other substances present in the composition.

The pharmaceutical composition and pharmaceutical dosage form according to the invention is well tolerated physiologically.

The pharmaceutical composition and pharmaceutical dosage form according to the invention provides Pomalidomide and optionally one or more other active pharmaceutical ingredients of a purity, uniformity and bioavailability level required for regulatory approval.

The pharmaceutical composition and pharmaceutical dosage form according to the invention can be prepared by an economical process suitable for use on an industrial scale. It has been surprisingly found that the pharmaceutical composition and the pharmaceutical dosage form according to the invention can be prepared by a simple technological process using simple blending of the ingredients and filling the thus obtained pharmaceutical composition (pharmaceutical mixture) into capsules and results in excellent physical and chemical stability, content uniformity and dissolution profile.

The pharmaceutical composition according to the invention is preferably used as an intermediate for the preparation of a pharmaceutical dosage form according to the invention.

The pharmaceutical dosage form according to the invention is an administration unit containing a therapeutic dose of Pomalidomide or a pharmaceutically acceptable salt thereof, which is contained in a defined amount of the pharmaceutical composition according to the invention. Besides a defined amount of the pharmaceutical composition according to the invention containing said therapeutic dose of Pomalidomide or a pharmaceutically acceptable salt thereof, the pharmaceutical dosage form according to the invention may contain additional elements, e.g. a coating or a capsule shell.

Unless expressly stated otherwise, all percentages are weight percent.

Further, unless expressly stated otherwise, all percentages refer to the total weight of the pharmaceutical composition (pharmaceutical mixture). The term *"pharmaceutical mixture"* as used herein designates the mixture comprising the active ingredient (i.e. Pomalidomide or a pharmaceutically acceptable salt thereof), the low substituted hydroxypropyl cellulose (L-HPC), and optionally the at least one pharmaceutically acceptable excipient. This pharmaceutical mixture (pharmaceutical composition) is preferably prepared to be further processed into the final pharmaceutical dosage form, such as compressed into a tablet, filled into a capsule, and the like. The term *"total weight of the pharmaceutical composition"* or *"total weight of the pharmaceutical mixture"* does not include the weight of the coating of the tablet or the weight of the capsule shell.

According to the invention and unless specified, all amount indications are provided on a weight basis or weight/weight basis, as appropriate. If Pomalidomide or any additional active pharmaceutical ingredient is present in the form of a pharmaceutically acceptable salt, the weight of the entire salt is to be considered, including the weight component of the counter ion. If Pomalidomide or any additional active pharmaceutical ingredient is present in the form of a solvate or a hydrate, the additional weight associated with the solvent or water components in the substance is to be disregarded. That is, a theoretical weight of the anhydrous pure substance (or its pharmaceutically acceptable salt and/or hydrate or solvate, if appropriate) is to be calculated and considered in connection with the invention.

In the pharmaceutical composition according to the invention, Pomalidomide or the physiologically acceptable salt thereof may be present in any known form or any polymorph forms known from the state of the art. Unless expressly stated otherwise, the term *"Pomalidomide"* as used herein refers to Pomalidomide in all forms.

The pharmaceutical composition according to the invention may contain Pomalidomide or a physiologically acceptable salt thereof in crystalline form or in amorphous form.

In preferred embodiments the pharmaceutical composition according to the invention comprises Pomalidomide or the physiologically acceptable salt thereof in predominately crystalline form.

Pomalidomide or the physiologically acceptable salt thereof in accordance with the invention may be characterized by particle size distribution (PSD) as determined by the laser diffraction method such as Malvern Master Sizer.

In preferred embodiments Pomalidomide or the physiologically acceptable salt thereof in the pharmaceutical composition according to the invention has a D(0.9) value less than 50 µm, preferably between 0.1 µm and 30 µm and more preferably between 1 µm and 20 µm.

In preferred embodiments Pomalidomide or the physiologically acceptable salt thereof in the pharmaceutical composition according to the invention has a D(0.5) value less than 20 µm, preferably less than 10 µm.

In preferred embodiments Pomalidomide or the physiologically acceptable salt thereof in the pharmaceutical composition according to the invention has a D(0.1) value less than 5 µm, preferably between 0.1 µm and 2 µm .

In preferred embodiments the amount of Pomalidomide or the physiologically acceptable salt thereof in the pharmaceutical composition according to the invention is between 0.05 wt.-% and 10 wt.-%, preferably between 0.1 wt.-% and 5 wt.-% and more preferably between 0.1 wt.-% and 3 wt.-%, based on the total weight of the pharmaceutical composition (pharmaceutical mixture).

In preferred embodiments the pharmaceutical composition according to the invention comprises
- Pomalidomide or a pharmaceutically acceptable salt thereof;
- one or more other active pharmaceutical ingredients; and
- a pharmaceutically acceptable excipient which is low substituted hydroxypropyl cellulose (L-HPC).

In preferred embodiments, the one or more other active pharmaceutical ingredients are selected from the group consisting of active ingredients having anticancer activity in humans such as a IgG1-derived monoclonal antibody that binds to a specific extracellular epitope of CD38 receptor, e.g. Daratumumab, Elotuzumab, Isatuximab, Dexamethasone, Prednisolone, Bortezomib, Cyclophosphamide; and proteasome inhibitors such as Carfilzomib, and Ixazomib.

While it is contemplated that the pharmaceutical composition according to the besides Pomalidomide or a pharmaceutically acceptable salt thereof may contain one or more additional active pharmaceutical ingredients, in other preferred embodiments Pomalidomide or the pharmaceutically acceptable salt thereof is the sole active pharmaceutical ingredients that is contained in the pharmaceutical composition according to the invention.

The pharmaceutical composition according to the invention comprises low substituted hydroxypropyl cellulose (L-HPC). The term *"low substituted hydroxypropyl cellulose"* as used herein can be abbreviated as L-HPC.

Low substituted hydroxypropyl cellulose (L-HPC) refers to a cellulose derivative having a degree of substitution with hydroxypropoxy-groups that does not exceed 20%, typically less than 15%, of the cellulose hydroxyl groups. Low-substituted hydroxypropyl cellulose is useful as a binder and as a disintegrant.

Low-substituted hydroxypropyl cellulose is commercially available in a number of different grades that have different particle sizes, shapes and substitution levels.

L-HPC is available by Shin-Etsu Chemical co. Ltd. e.g. as type LH-11, LH-21, LH-22, LH-31, LH-32, LH-B1, NBD-022, NBD-021, and NBD-020. The types differ in particle size distribution, characterized by mean particle size and 90% cumulated particle size, and degree of substitution, characterized by hydroxypropoxy content (%), these characteristics are shown in the table here below:

| Grade | Hydroxypropoxy content (%) | Mean particle size (µm) | 90 % cumulated particle size(µm) |
|---|---|---|---|
| LH-11 | 11 | 55 | 175 |
| LH-21 | 11 | 45 | 135 |
| LH-22 | 8 | 45 | 135 |
| LH-B1 | 11 | 55 | 125 |
| LH-31 | 11 | 20 | 70 |
| LH-32 | 8 | 20 | 70 |
| NBD-022 | 8 | 45 | 100 |
| NBD-021 | 11 | 45 | 100 |
| NBD-020 | 14 | 45 | 100 |

Preferably, the L-HPC has a degree of substitution with hydroxypropoxy-groups of 8 to 11 % and 90 % cumulated particle size in the range of 100 to 150 µm.

Preferably, the L-HPC is according to USP or according to Ph. Eur. in the official version valid on January 1, 2023.

The amount of L-HPC in the pharmaceutical composition is preferably at least 30 wt.-%, preferably at least 35 wt.-%, more preferably at least 40 wt.-%, based on the total weight of the pharmaceutical composition.

The amount of L-HPC in the pharmaceutical composition is typically in the range of about 30 wt.-% to 99.9 wt.-% of the pharmaceutical composition. Preferably, the amount of L-HPC is 40 wt.-% to 80 wt.-%, especially 40 wt.-% to 70 wt.-%, based on the total weight of the pharmaceutical composition (pharmaceutical mixture).

The L-HPC is preferably present in intimate admixture with the Pomalidomide or the physiologically acceptable salt thereof. The Pomalidomide or the physiologically acceptable salt thereof is preferably directly exposed to the L-HPC. Thus, the L-HPC and the Pomalidomide or the physiologically acceptable salt thereof are preferably not contained in separate phases of the pharmaceutical composition, but as a mixture, preferably a powder mixture.

The pharmaceutical composition, preferably for oral administration, according to the invention comprises Pomalidomide or a physiologically acceptable salt thereof, L-HPC and optionally at least one pharmaceutically acceptable excipient selected from the group comprising at least one binder, at least one diluent, at least one disintegrant, at least one glidant and at least one lubricant.

Preferably, the at least one pharmaceutically acceptable excipient comprises
- a diluent selected from starch or sugar alcohol;
- optionally, one or more further diluents,
- optionally, at least one disintegrant,
- optionally, at least one glidant, and
- optionally, at least one lubricant.

Binders, diluents, disintegrants, glidants and lubricants are known to the skilled person. In this regard, reference can be made to e.g. R. Rowe et al., Handbook of Pharmaceutical Excipients, 9th ed. 2020.

The pharmaceutical composition according to the invention may comprise one or more diluents.

Diluents are preferably selected from
- polysaccharides (e.g. starches),
- monosaccharides,
- disaccharides,
- oligosaccharides,
- sugar alcohols, and
- mixtures thereof.

Starch may be selected from partially or wholly pregelatinized starch, corn starch, wheat starch, rice starch, tapioca starch, potato starch and any mixture thereof.

Monosaccharides, disaccharides, oligosaccharides and sugar alcohols may be selected from glucose, fructose, sucrose, raffinose, isomaltose, trehalose, dextrates, mannitol, erythritol, sorbitol, isomalt, maltitol, xylitol, lactitol, compressible sugars, and mixtures thereof.

Preferably, the diluent is selected from starch, pregelatinized starch, mannitol, maltitol, lactitol and isomalt.

Preferably the diluent is not selected from lactose, calcium salts of phosphoric acid or carbonic acid or sulfuric acid, such as calcium hydrogen phosphate anhydrous or hydrate or calcium carbonate.

It has been surprisingly found that a particular excipients combination, i.e. a mixture of low substituted hydroxyl propyl cellulose (L-HPC) and a diluent, provides for a Pomalidomide pharmaceutical composition showing desired chemical stability, dose uniformity and dissolution profile.

It was found that the optimal results are achieved by using a specific ratio of cellulose L-HPC and diluent. The diluent is preferably selected from starch, pregelatinized starch or sugar alcohol selected from the group of mannitol, lactitol, isomalt, sorbitol and maltitol. The combination of L-HPC with isomalt is particularly preferred.

The typical weight ratio of L-HPC to diluent in a pharmaceutical composition according to the invention can be from 100:1 to 0.5:1, preferably 20:1 to 0.5:1, more preferably from 5:1 to 1:1.

The diluents are present in an amount preferably ranging from about 0.5 wt.-% to about 50 wt.-%, preferably 10 wt.-% to 50 wt.-%, more preferably 20 wt.-% to 50 wt.-% of the total weight of the pharmaceutical composition (pharmaceutical mixture).

Preferably, the combined total amount of L-HPC and the one or more diluents, preferably isomalt, mannitol and/or pregelatinized starch, is at least 70 wt.-%, preferably at least 72.5 wt.-%, more preferably at least 75 wt.-%, still more preferably at least 77.5 wt.-%, yet more preferably at least 80 wt.-%, even more preferably at least 82.5 wt.-%, most preferably at least 85 wt.-%, and in particular at least 87.5 wt.-% of the total weight of the pharmaceutical composition (pharmaceutical mixture).

The pharmaceutical composition according to the invention may comprise one or more disintegrants.

Disintegrants are preferably selected from, but not limited to, crospovidone, starch, maize starch, pregelatinized starch, sodium starch glycollate, modified starch, hydroxypropyl starch, carboxymethyl starch, sodium and/or calcium salts of carboxymethyl cellulose, cross-linked carboxymethylcellulose (e.g. croscarmellose sodium and/or croscarmellose calcium), polacrilin potassium, alginic acid or alginates, sodium and/or calcium alginate, polyacrylates, docusate sodium, methylcellulose, agar, guar gum, chitosan, gums and mixtures thereof.

Disintegrants are preferably selected from crospovidone, starch, maize starch, pregelatinized starch, modified starch, hydroxypropyl starch or carboxymethyl starch.

The disintegrants are present in an amount preferably ranging from about 0.5 wt.-% to about 25 wt.-%, preferably 0.5 wt.-% to 15 wt.-%, more preferably 1 wt.-% to 10 wt.-% of the total weight of the pharmaceutical composition (pharmaceutical mixture).

The pharmaceutical composition according to the invention may comprise one or more lubricants.

Lubricants are preferably selected from, but not limited to,
- fatty acids (i.e. carboxylic acids with 12 to 20 carbon atoms);
- fatty acid esters including glyceride esters such as glyceryl monostearate, glyceryl tribehenate, or glyceryl dibehenate (e.g. Compritol^{®} 888);
- metal salts of fatty acids, including magnesium, calcium, aluminum or zinc salts of fatty acids (e.g. magnesium, calcium, aluminum or zinc stearate, magnesium palmitate, or magnesium oleate);
- hydrogenated vegetable oil, hydrogenated castor oil;
- waxes (e.g. Sterotex^{®} NF, Lubriwax^{®} [hydrogenated vegetable oil type], meads wax or spermaceti);
- boric acid;
- sodium stearyl fumarate;
- polymers (e.g., PEG, macrogols);
- sugar esters such as sorbitan monostearate and sucrose monopalmitate and
- any mixtures thereof.

Lubricants are preferably selected from fatty acids, fatty acid esters including glyceryl tribehenate, or glyceryl dibehenate, hydrogenated vegetable oil, hydrogenated castor oil, sodium stearyl fumarate or polymers.

The lubricants are present in an amount preferably ranging from about 0.025 wt.-% to about 5 wt.-%, preferably 0.05 wt.-% to 3.0 wt.-%, more preferably 0.1 wt.-% to 2.0 wt.-% of the total weight of the pharmaceutical composition (pharmaceutical mixture).

The pharmaceutical composition according to the invention may comprise one or more glidants.

Glidants are preferably selected from, but not limited to, colloidal silicon dioxide (colloidal silica), talc, magnesium trisilicate, and mixtures thereof.

Preferred glidants are colloidal silica, talc and any mixtures thereof.

The glidants are present in an amount preferably ranging from about 0.0 wt.-% to about 5.0 wt.-%, preferably 0.0 wt.-% to 3.0 wt.-%, more preferably 0 wt.-% to 2 wt.-% of the total weight of the pharmaceutical composition (pharmaceutical mixture).

Preferably, the pharmaceutical composition is substantially free of lactose, microcrystalline cellulose and calcium hydrogen phosphate; more preferably, the pharmaceutical composition is free of any lactose, any microcrystalline cellulose and any calcium hydrogen phosphate.

Preferably, the pharmaceutical composition is free of calcium salts of phosphoric acid or carbonic acid or sulfuric acid, such as calcium hydrogen phosphate anhydrous or hydrate or calcium carbonate.

In particular, the following items are provided herein:
Item 1. A pharmaceutical composition, preferably for oral administration, comprising Pomalidomide and L-HPC and optionally at least one pharmaceutically acceptable excipient selected from the group comprising at least one binder, at least one diluent, at least one disintegrant, at least one glidant and at least one lubricant.
Item 2. The pharmaceutical composition according to item 1 comprising
   - 0.1 wt.-% to 3 wt.-% of Pomalidomide or a pharmaceutically acceptable salt thereof in crystalline form or amorphous form;
   - 30 wt.-%% to 99.9 wt.-% of L-HPC;
      and optionally
   - 10 wt.-% to 50 wt.-% of one or more diluents;
   - 1 wt.-% to 10 wt.-% of one or more disintegrants; and
   - 0.1 wt.-% to 3 wt.-% of one or more lubricants;
      based on the total weight of the pharmaceutical composition (pharmaceutical mixture).

In preferred embodiments, the pharmaceutical composition comprises or essentially consists of
- Pomalidomide of a physiologically acceptable salt thereof,
- L-HPC,
- crospovidone,
- a diluent selected from isomalt, mannitol and pregelatinized starch; and
- a lubricant selected from sodium stearyl fumarate and glyceryl behenate.

The above-mentioned components of the pharmaceutical composition according to the invention may be present in relative amounts as shown in the following table. Amount indications in the following tables may be understood as indications in parts by weight of the pharmaceutical composition (pharmaceutical mixture):

| [wt.-%] | Embodiment 1 | Embodiment 2 | Embodiment 3 |
|---|---|---|---|
| Pomalidomide | 0.5 to 10 | 0.1 to 5 | 0.1 to 3 |
| L-HPC | 30 to 99.9 | 40 to 80 | 40 to 70 |
| diluent | 0.5 to 50 | 10 to 50 | 20 to 50 |
| disintegrant | 0.5 to 25 | 0.5 to 15 | 1 to 10 |
| glidant | 0 to 20 | 0 to 5 | 0 to 2 |
| lubricant | 0.025 to 5 | 0.05 to 3 | 0.1 to 2 |

In preferred embodiments the pharmaceutical composition according to the comprises
- 0.1 wt.-% to 3 wt.-% of Pomalidomide or a pharmaceutically acceptable salt thereof in crystalline form or amorphous form;
- 30 wt.-% to 99.9 wt.-% of L-HPC;
   and optionally
- 10 wt.-% to 50 wt.-% of one or more diluents;
- 1 wt.-% to 10 wt.-% of one or more disintegrants; and
- 0.1 wt.-% to 2 wt.-% of one or more lubricants;
   based on the total weight of the pharmaceutical composition (pharmaceutical mixture).

In preferred embodiments the pharmaceutical composition according to the invention comprises
- 0.1 wt.-% to 3 wt.-% of Pomalidomide or a pharmaceutically acceptable salt thereof in crystalline form or amorphous form;
- 30 wt.-% to 99.9 wt.-% of L-HPC;
- 10 wt.-% to 50 wt.-% of one or more diluents;
   and optionally
- 1 wt.-% to 10 wt.-% of one or more disintegrants; and
- 0.1 wt.-% to 2 wt.-% of one or more lubricants;
   based on the total weight of the pharmaceutical composition (pharmaceutical mixture).

In preferred embodiments the pharmaceutical composition according to the invention comprises
- 0.1 wt.-% to 3 wt.-% of Pomalidomide or a pharmaceutically acceptable salt thereof in crystalline form or amorphous form;
- 30 wt.-% to 99.9 wt.-% of L-HPC;
- 10 wt.-% to 50 wt.-% of one or more diluents;
- 1 wt.-% to 10 wt.-% of one or more disintegrants;
   and optionally
- 0.1 wt.-% to 2 wt.-% of one or more lubricants;
   based on the total weight of the pharmaceutical composition (pharmaceutical mixture).

In preferred embodiments the pharmaceutical composition according to the invention comprises
- 0.1 wt.-% to 3 wt.-% of Pomalidomide or a pharmaceutically acceptable salt thereof in crystalline form or amorphous form;
- 30 wt.-% to 99.9 wt.-% of L-HPC;
- 10 wt.-% to 50 wt.-% of one or more diluents;
- 1 wt.-% to 10 wt.-% of one or more disintegrants; and
- 0.1 wt.-% to 2 wt.-% of one or more lubricants;
   based on the total weight of the pharmaceutical composition (pharmaceutical mixture).

In preferred embodiments the pharmaceutical composition according to the invention comprises
- 0.1 wt.-% to 3 wt.-% of Pomalidomide or a pharmaceutically acceptable salt thereof in crystalline form or amorphous form;
- 40 wt.-% to 95 wt.-%, preferably 40 to 50 wt.-% of L-HPC;
- 10 to 50 wt.-%, preferably 40 to 50 wt.-% of one or more diluents; preferably selected from isomalt, mannitol, and pregelatinized starch;
- 1 wt.-% to 10 wt.-%, preferably 5 wt.-% to 10 wt.-% of one or more disintegrants; preferably crospovidone; and
- 0.1 wt.-% to 2 wt.-%, preferably 0.1 wt.-% to 1 wt.-% of one or more lubricants; preferably sodium stearyl fumarate or glyceryl behenate;
   based on the total weight of the pharmaceutical composition (pharmaceutical mixture).

Another aspect of the invention relates to a process for manufacturing the pharmaceutical composition according to the invention as described above, said process comprising the following steps:
- mixing Pomalidomide or a physiologically acceptable salt thereof, L-HPC and at least one excipient or a mixture of excipients to form a powder mixture which can be optionally sieved before mixing to give a mixture;
- sieving the mixture, optionally addition of further excipients or mixture of excipients; and
- optionally, blending the mixture.

According to preferred embodiments, the process for manufacturing the pharmaceutical composition according to the invention comprises the following steps:
- mixing Pomalidomide or a physiologically acceptable salt thereof, a portion of L-HPC, and optionally a diluent in a weight/weight ratio from 1:1 to 1: 50, preferably from 1:1.5 to 1:30, more preferably from 1:2 to 1:25, and/or further excipients and sieving the mixture;
- mixing the above obtained mixture with another portion of L-HPC and diluent and/or further excipients in a mixer to obtain homogenized mixture;
- optionally dry sieving the mixture through a sieve in order to segregate cohesive particles and to improve content uniformity; and
- adding lubricant and/or glidant and optionally the remaining portion of L-HPC and mixing the mixture to obtain a mixture.

Another aspect of the invention relates to a pharmaceutical dosage form for oral administration comprising the pharmaceutical composition according to the invention.

The pharmaceutical dosage form according to the invention is preferably a solid dosage form. Preferred solid pharmaceutical dosage forms according to the invention include, but are not limited to, capsules, powders, tablets, minitablets, microtablets, coated or uncoated tablets, coated or uncoated minitablets, coated or uncoated microtablets, coated or uncoated multilayer tablets, coated or uncoated bilayer tablets, coated pellets, pills, lozenges, and the like.

In preferred embodiments, the pharmaceutical dosage form according to the invention is a capsule. The term capsule as used herein encompasses capsules of all shapes and sizes. The capsules may be selected from the group comprising hard gelatin capsules, gelatin/PEG capsules, pullulan capsules, PVA capsules, starch capsules and hard hydroxypropyl methylcellulose or hypromellose (HPMC) capsules, preferably hard gelatin, hard hydroxypropyl methylcellulose or hypromellose (HPMC) capsules.

In other preferred embodiments, the pharmaceutical dosage form according to the invention is a tablet.

For the production of pharmaceutical dosage forms (e.g. capsules or tablets) according to the invention it is preferred that the particle size of all excipients is of the same particle size range in order to minimize the segregation of the component of the mixture. The average particle sizes (especially weight average sizes) of the excipients used are preferably independently of one another in the range of 20 to 200 µm, more preferably in the range of 50 to 150 µm. The main advantages of the use of the excipients with a particle size of the same degree are a high content uniformity due to a low segregation tendency, and a good flowability providing a high weight consistency at various capsule filling speeds.

The pharmaceutical dosage form according to the invention is preferably suitable and intended for oral administration, preferably for per oral administration.

The oral pharmaceutical dosage form according to the invention preferably comprise from 1 mg to 4 mg of Pomalidomide or of a physiologically acceptable salt thereof, such as 1 mg, 2 mg, 3 mg, or 4 mg. The qualitative composition is preferably the same for dose strength of 1 mg, 2 mg, 3 mg and 4 mg.

The pharmaceutical dosage form according to the invention provides immediate release (conventional release) of the active ingredient(s) present therein.

Another aspect of the invention relates to a process for manufacturing the pharmaceutical dosage form according to the invention as described above.

In preferred embodiments, e.g. when the pharmaceutical dosage form is a capsule, the process comprises the step of filling a predetermined amount of the pharmaceutical composition according to the invention into a capsule.

In other preferred embodiments, e.g. when the pharmaceutical dosage form is a tablet, the process comprises the step of compressing a predetermined amount of the pharmaceutical composition according to the invention into a tablet, and optionally film-coating the tablet.

Preferably, the process for manufacturing the pharmaceutical dosage form according to the invention comprises the process for manufacturing the pharmaceutical composition according to the invention as described above.

In preferred embodiments, the pharmaceutical dosage form according to the invention may be administered in combination with at least one additional active pharmaceutical ingredient selected from, but not limited to, active ingredients having anticancer activity in humans such as a IgG1-derived monoclonal antibody that binds to a specific extracellular epitope of CD38 receptor, e.g. Daratumumab, Elotuzumab, Isatuximab, Dexamethasone, Prednisolone, Bortezomib, Cyclophosphamide; and proteasome inhibitors e.g. Carfilzomib, or Ixazomib.

Said at least one additional active pharmaceutical ingredient may be contained in the pharmaceutical dosage form according to the invention or in a separate pharmaceutical dosage form.

The pharmaceutical dosage form according to the invention, preferably oral pharmaceutical dosage form, is particularly suitable for use in the treatment of cancer, disorders associated with angiogenesis, pain including, but not limited to, Complex Regional Pain Syndrome ("CRPS"), Macular Degeneration ("MD") and related syndromes, skin diseases, pulmonary disorders, asbestos-related disorders, parasitic diseases, immunodeficiency disorders, CNS disorders, CNS injury, atherosclerosis and related disorders, dysfunctional sleep and related disorders, hemoglobinopathy and related disorders (e.g., anemia), TNFα related disorders, and other various diseases and disorders, as described in detail in, e.g., WO 2010 135396 .

In another embodiment the pharmaceutical dosage form according to the invention, preferably oral pharmaceutical dosage form comprising the pharmaceutical composition is suitable for use in treatment of relapsed and refractory multiple myeloma.

Preferred specific embodiments according to the invention are described in the following examples. It is, however, to be understood that the invention is not limited to these examples.

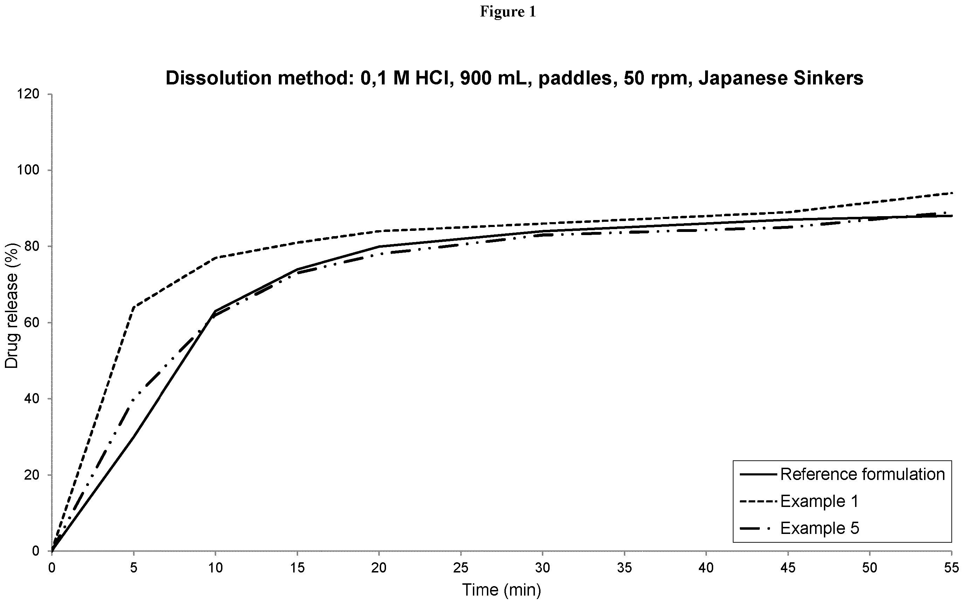

Figure 1 shows dissolution profile of the capsules comprising Pomalidomide prepared according to Examples 1 and 5 and dissolution profile of the reference drug Imnovid^{®} available on the market.

### Methods

### Related substances/Impurities of Pomalidomide by HPLC

The analytical method used for determining impurities of Pomalidomide was gradient HPLC method, using YMC-Pack Pro column with stationary phase C18, with UV detection at wavelength 240 nm. The mobile phase was a combination of potassium dihydrogenphosphate solution (pH=2.5) and methanol/acetonitrile mixture.

### Dissolution profile

Dissolution profiles (especially for determining immediate release) can be determined by a dissolution test performed on the compositions (capsules) to be tested according to Chapter 711 (Dissolution) of US Pharmacopeia (e.g. as in force on the priority date of the present application), especially using Apparatus 2 in combination with Japanese Basket Sinkers :

Dissolution method for Pomalidomide: Apparatus 2 using Japanese Basket Sinkers as described in Chapter 711 (Dissolution) of the US Pharmacopeia under the following conditions: Stirring speed: 50 rpm, Temperature: 37°C, Dissolution medium: 0.1 M Hydrochloric acid, Medium volume: 900 ml, dissolution time: 50 min, sampling interval: 5 min.

The dissolution profiles shown in Figure 1 was determined as indicated supra, at a temperature: 37°C (± 0,5°C). To establish the dissolution profile aliquots were taken at regular intervals of 5 minutes from the dissolution vessels and the concentration of the drug therein was determined by HPLC.

The content uniformity of the tablets is characterized by the relative standard deviation (RSD) and Acceptance Value (AV), which are determined according to standard procedures as described in European Pharmacopoeia (2.9.40. Uniformity of Dosage Units). The test for content uniformity of preparations presented in dosage units is based on the assay of the individual contents of active substance of a number of dosage units.

### Examples 1-4

| [wt.-%] | Example 1 | Example 2 | Example 3 | Example 4 |
|---|---|---|---|---|
| Pomalidomide | 2.04 | 2.04 | 2.04 | 2.04 |
| L-HPC | 44.89 | 44.89 | 44.89 | 64.89 |
| Isomalt | 44.89 | / | 44.89 | 24.89 |
| Pregelatinized Starch | / | 44.89 | / | / |
| Crospovidone | 7.85 | 7.85 | 7.85 | 7.85 |
| Sodium stearyl fumarate | 0.33 | 0.33 | / | 0.33 |
| Compritol^{®} 888 | / | / | 0.33 | / |

### Manufacturing process

Pomalidomide is mixed with a portion of L-HPC and isomalt or pregelatinized starch to obtain a homogenous triturate. The prepared triturate is sieved. The remaining quantity of isomalt or pregelatinized starch, second part of L-HPC and crospovidone are sieved, added to triturate and mixed to obtain a homogenous mixture. Sodium stearyl fumarate or Compritol^{®} 888 are mixed with the remaining quantity of L-HPC, added to the mixture and mixed to prepare a homogenized capsulation mixture. The capsulation mixture is filled into capsules to prepare the final solid pharmaceutical dosage form.

Dissolution profile result for Example 1 in comparison to the reference product is shown in Figure 1.

Chemical stability results for Example 1 are shown in the table below:

| Sample | Condition | Related substances - individual | Related substances - total |
|---|---|---|---|
| Example 1 | 40 °C/75 % RH 1 month, closed | ≤0.10 % | ≤0.10 % |
| | 45 °C/75 % RH 1 month, closed | ≤0.10 % | ≤0.10 % |

Content uniformity (Acceptance value in %) result for Example 1 is 4.5 %.

Pharmaceutical dosage form according to example 1 is bioequivalent to the reference product Imnovid, as confirmed in single-dose bioequivalence study under fasting conditions. All the tested parameters were found completely within the 80-125% bioequivalence limits.

### Examples 5-8

| [wt.-%] | Example 5 | Example 6 | Example 7 | Example 8 |
|---|---|---|---|---|
| Pomalidomide | 2.21 | 2.21 | 2.21 | 2.21 |
| L-HPC | 44.61 | 44.61 | 64.61 | 89.61 |
| Mannitol | 45.00 | 45.00 | 25.00 | / |
| Crospovidone | 7.85 | 7.85 | 7.85 | 7.85 |
| Sodium stearyl fumarate | 0.33 | / | 0.33 | 0.33 |
| Compritol^{®} 888 | / | 0.33 | / | / |

### Manufacturing process

Pomalidomide is mixed with a portion of L-HPC and optionally mannitol to obtain a homogenous triturate. The prepared triturate is sieved. The remaining quantity of mannitol, second part of L-HPC and crospovidone are sieved, added to triturate and mixed to obtain a homogenous mixture. Sodium stearyl fumarate or Compritol^{®} 888 are mixed with the remaining quantity of L-HPC, added to the mixture and mixed to prepare a homogenized capsulation mixture. The capsulation mixture is filled into capsules to prepare the final solid pharmaceutical dosage form.

Dissolution profile result for Example 5 in comparison to the reference product is shown in the Figure 1.

Chemical stability results for Example 5 are shown in the table below:

| Sample | Condition | Related substances - individual | Related substances - total |
|---|---|---|---|
| Example 5 | 40 °C/75 % RH 1 month, closed | ≤0.10 % | ≤0.10 % |
| | 45 °C/75 % RH 1 month, closed | ≤0.10 % | ≤0.10 % |

Content uniformity (Acceptance value in %) result for Example 5 is 9.5 %.

### Examples 9 - 11

Different product strengths, i.e. 3 mg, 2 mg and 1 mg can be derived from above presented examples, which relate to the pharmaceutical dosage form of 4 mg product strength. Change in the dosage strength results in adjustment of Pomalidomide and diluent quantities. Below examples show compositions of different strengths prepared based on above presented Example 1.

| [wt.-%] | Example 9 | Example 10 | Example 11 |
|---|---|---|---|
| Pomalidomide | 1.53 | 1.02 | 0.51 |
| L-HPC | 44.89 | 44.89 | 44.89 |
| Isomalt | 45.40 | 45.91 | 46.42 |
| Crospovidone | 7.85 | 7.85 | 7.85 |
| Sodium stearyl fumarate | 0.33 | 0.33 | 0.33 |

## Claims

1. A pharmaceutical composition, preferably for oral administration, comprising
- Pomalidomide or a pharmaceutically acceptable salt thereof;
- low substituted hydroxypropyl cellulose (L-HPC); and
- optionally, at least one pharmaceutically acceptable excipient selected from the group consisting of binders, diluents, disintegrants, glidants and lubricants.

2. The pharmaceutical composition according to claim 1, wherein the Pomalidomide or the pharmaceutically acceptable salt thereof is predominantly crystalline.

3. The pharmaceutical composition according to claim 1 or 2, wherein the amount of L-HPC is at least 30 wt.-%, preferably at least 35 wt.-%, more preferably at least 40 wt.-%, based on the total weight of the pharmaceutical composition.

4. The pharmaceutical composition according to any of the preceding claims, which comprises
- 0.1 to 3 wt.-% of Pomalidomide or a pharmaceutically acceptable salt thereof;
- 30 wt.-% to 99.9 wt.-% of L-HPC;
- optionally, 10 wt.-% to 50 wt.-% of one or more diluents;
- optionally, 1 wt.-% to 10 wt.-% of one or more disintegrants; and
- optionally, 0.1 wt.-% to 2 wt.-% of one or more lubricants;
based on the total weight of the pharmaceutical composition.

5. The pharmaceutical composition according to any of the preceding claims, which comprises
- 0.1 to 3 wt.-% of Pomalidomide or a pharmaceutically acceptable salt thereof;
- 30 wt.-% to 99.9 wt.-% of L-HPC;
- 10 wt.-% to 50 wt.-% of one or more diluents;
- optionally, 1 wt.-% to 10 wt.-% of one or more disintegrants; and
- optionally, 0.1 wt.-% to 2 wt.-% of one or more lubricants;
based on the total weight of the pharmaceutical composition.

6. The pharmaceutical composition according to any of the preceding claims, which comprises
- 0.1 wt.-% to 3 wt.-% of Pomalidomide or a pharmaceutically acceptable salt thereof; preferably in crystalline form or amorphous form;
- 30 wt.-% to 99.9 wt.-% of L-HPC;
- 10 wt.-% to 50 wt.-% of one or more diluents;
- 1 wt.-% to 10 wt.-% of one or more disintegrants; and
- optionally, 0.1 wt.-% to 2 wt.-% of one or more lubricants;
based on the total weight of the pharmaceutical composition.

7. The pharmaceutical composition according to any of the preceding claims, which comprises
- 0.1 wt.-% to 3 wt.-% of Pomalidomide or a pharmaceutically acceptable salt thereof; preferably in crystalline form or amorphous form;
- 30 wt.-% to 99.9 wt.-% of L-HPC;
- 10 wt.-% to 50 wt.-% of one or more diluents;
- 1 wt.-% to 10 wt.-% of one or more disintegrants; and
- 0.1 wt.-% to 2 wt.-% of one or more lubricants;
based on the total weight of the pharmaceutical composition.

8. The pharmaceutical composition according to any of the preceding claims, wherein the one or more diluents are selected from (i) polysaccharides (e.g. starches), (ii) monosaccharides, (iii) disaccharides, (iv) oligosaccharides, (v) sugar alcohols, and mixtures thereof; preferably from the mannitol, lactitol, isomalt, sorbitol and maltitol.

9. The pharmaceutical composition according to any of the preceding claims, wherein the weight ratio of L-HPC to diluent is from 100:1 to 0.5:1, preferably from 20:1 to 0.5:1, more preferably from 5:1 to 1:1.

10. The pharmaceutical composition according to any of the preceding claims, wherein the one or more disintegrants are selected from crospovidone, starch, maize starch, pregelatinized starch, sodium starch glycollate, modified starch, hydroxypropyl starch, carboxymethyl starch, sodium and/or calcium salts of carboxymethyl cellulose, cross-linked carboxymethylcellulose and salts thereof, polacrilin potassium, alginic acid or alginates, sodium and/or calcium alginate, polyacrylates, docusate sodium, methylcellulose, agar, guar gum, chitosan, gums and mixtures thereof.

11. The pharmaceutical composition according to any of the preceding claims, wherein the one or more lubricants are selected from (i) fatty acids; (ii) fatty acid esters including glyceride esters; (iii) metal salts of fatty acids; (iv) hydrogenated vegetable oil, hydrogenated castor oil; (v) waxes; (vi) boric acid; (vii) sodium stearyl fumarate; (viii) polymers; (ix) sugar esters, and mixtures thereof.

12. The pharmaceutical composition according to any of the preceding claims, which is substantially free of lactose, microcrystalline cellulose and calcium hydrogen phosphate.

13. A pharmaceutical dosage form for oral administration comprising the pharmaceutical composition according to any of the preceding claims.

14. The pharmaceutical dosage form according to claim 13, which is a filled capsule.
